# EUROPEAN PATENT SPECIFICATION

(11) **EP 1 202 758 B1**
(45) Date of publication and mention of the grant of the patent: **30.08.2006**
(21) Application number: 00945212.9
(22) Date of filing: 07.07.2000
(51) Int. Cl.: A61M 1/02, A61M 1/36, B01L 3/14

(54) **PLATELET CONCENTRATION SYRINGE KIT AND METHOD**
SPRITZENKIT FÜR DIE KONZENTRATION VON THROMBOZYTEN UND VERFAHREN
TROUSSE DE SERINGUES A CONCENTRATION DE PLAQUETTES ET PROCEDURE

(30) Priority: 08.07.1999 US 142886 P
(43) Date of publication of application: 08.05.2002
(73) Proprietor: IMPLANT INNOVATIONS, INC., Palm Beach Gardens, Florida 33410 (US)
(72) Inventor: JORGENSEN, Glen, E., Marlboro, MA 01752 (US); BERCKMANS, Bruce, III, Palm Beach Gardens, FL 33410 (US)
(74) Representative: Grünecker, Kinkeldey, Stockmair & Schwanhäusser Anwaltssozietät
(86) International application number: PCT/US2000/018553
(87) International publication number: WO 2001/003756

(56) References cited:
- WO-A-91/17778
- DE-A- 4 445 030
- US-A- 5 858 253

## Description

### Field of the Invention

This invention relates to processing whole blood into fractions and, more particularly, to improvements in blood processing systems for separating platelet-rich plasma from autologous blood.

### Background of the Invention

The science and effectiveness of using platelet-rich plasma derived from the patient's own blood in surgery are documented in medical, trade, and science journals. A known method for the preparation of platelets from whole blood is described in the American Association of Blood Bank's Technical Manual, 12th Edition, 1996, at pages 700-701, Method 9.11. A system employing this method collects the patient's whole blood into a collection unit with two integrally attached transfer containers. The blood is collected into the collecting container, the other two transfer containers are collapsed, and the two transfer containers with the collecting container are subjected to a "soft spin" in a centrifuge, which brings the plasma to the top of the collecting unit, leaving red cells at the bottom. In the next step, the collecting container containing the blood fractions are squeezed in a plasma extractor to force the platelet-rich plasma into one of the transfer containers through a connecting tube. A fraction comprising red cells remains behind in the collecting container, which is then removed. Next, the two transfer containers, the first being empty and the second containing the plasma, are subjected to a "heavy spin" in a centrifuge to concentrate platelets at the "bottom" of the second transfer container, leaving a platelet-poor fraction of the plasma (PPP) above the platelet concentrate (PC) in the second transfer container. The following step squeezes the second transfer container to express the PPP into the first transfer container. The platelet concentrate (PC) is then re-suspended and collected for use. This system uses a process requiring six separate steps, including two centrifugal steps and two separation steps. The terms "light spin" and "heavy spin" are defined in Table 10.5-1 at page 716 of the AABB Technical Manual.

An alternative method of separating blood fractions is discussed in U.S. Patent No. 5,102,407 and referred to as the "Amsterdam" method. Instead of two spins, soft then hard, as discussed above, a single hard spin is used in which three fractions are separated, that is, a relatively platelet-free plasma, red blood cells, and an intermediate "buffy coat" layer which contains platelets and leukocytes.

A medical apparatus configured to be used in the centrifuge is described in DE 44 45 030 A1. The apparatus consists of a cylindrical hollow body, having a conical reducing end, that fits a standard centrifuge. A plunger is moveably engageable with the interior surface of the body. The plunger comprises a central bore and is removable engaged to a plunger rod. The central bore and a central bore in the plunger rod form a passage from the interior of the body to the free end of the plunger rod. The plunger of the plunger rod can be closed with a cap while used in centrifuge.

The usual procedures often employ pliable plastic bags which make it difficult to separate the blood fractions accurately. Consequently, improvements have been needed. The present invention is embodied in a new blood separation kit which provides a more convenient and efficient means for separating a patient's blood and in particular for recovering platelets.

### Summary of the Invention

The invention provides a kit according to claim 1 and a method according to claims 4 or 5. In one embodiment, the system is in the form of a syringe kit including disposable components supplied in sterile disposable packaging, and having all of the components required to draw blood from the patient, prevent blood coagulation, separate the sample into fractions including platelet concentrate, and deliver the platelet concentrate to a surgical site. More precise separation of the separated blood fractions is possible, compared to the results achieved with pliable plastic bags. Such a system will be useful to medical and dental practitioners.

The invention may be generally described as including an elongated container, preferably cylindrical, which is adapted to be placed in a centrifuge for separating the fractions of blood introduced into the container. The container is equipped with a first port through which the blood fractions can be expelled by a plunger movably fitted within the container. The first port is located at a first end of the container and a second port is located on the movable plunger. Both ports are normally closed and they are opened from outside the container as required for removal of blood fractions. The plunger is equipped with a detachable hollow plunger rod adapted to open the second port when the plunger rod is attached to the plunger. A third port is mounted at the end of the plunger rod, the third port also is normally closed unless opened from outside the plunger rod.

In one embodiment of the invention, the container is in the form of a syringe barrel fitted with a moveable plunger. The syringe barrel is charged with a patient's blood and all of the centrifugal and separation steps are performed while the blood is in that container. The syringe barrel is adapted for use in a centrifuge, and has valves fitted at one end and within the plunger, making it possible to collect blood, centrifuge the blood in the syringe barrel, expel red blood cells after the first or soft spin, centrifuge the remaining platelet-rich plasma and expel platelet-poor plasma after the second or hard spin. The platelet concentrate (PC) remains in the syringe barrel, where it can be re-suspended in a medium or media that can be introduced into the container through one of the valves.

In another embodiment, the container of the invention is given a "hard" spin, omitting the "soft" spin. Three fractions are formed, the first fraction, the red blood cells, is expelled through the first port, then the second traction, platelet-poor plasma, is expelled through the second and third ports, leaving the third layer, consisting of platelets and plasma, for further processing.

The separation of blood fractions may be carried out manually after centrifuging the whole blood in the container. Alternatively, the separation of blood fractions could be carried out automatically in centrifuge equipment having facilities for opening the ports after it has been determined that the desired separation has been made.

Associated with the container preferably will be a needle assembly for drawing a patient's blood and which is adapted to transfer the blood into the syringe container directly or into a separate container for subsequent transfer into the syringe container. One or more waste bags adapted to receive platelet-poor plasma and red blood cells from the syringe cylinder after the soft and hard spins may be part of the syringe kit.

### Brief Description of the Drawings

FIG. 1 is an isometric view of a elongated syringe container and associated components.
FIG. 2 is a longitudinal section through FIG. 1.
FIG. 3 shows a blood-collector needle.
FIG. 4 shows a waste bag.
FIGS. 5a through 5e show use of the syringe embodiment to prepare platelet concentrate.

### Description of the Illustrative Embodiments

The figures illustrate a syringe embodiment of the invention adapted for manual handling in separation of blood fractions. Blood drawn from a patient is introduced into a syringe, the syringe is placed in a centrifuge and spun at the appropriate speed for a suitable period of time to cause the blood to be separated into at least two fractions. Alternatively, the separation may be automated and the blood fractions separated without manual handling of the syringe container.

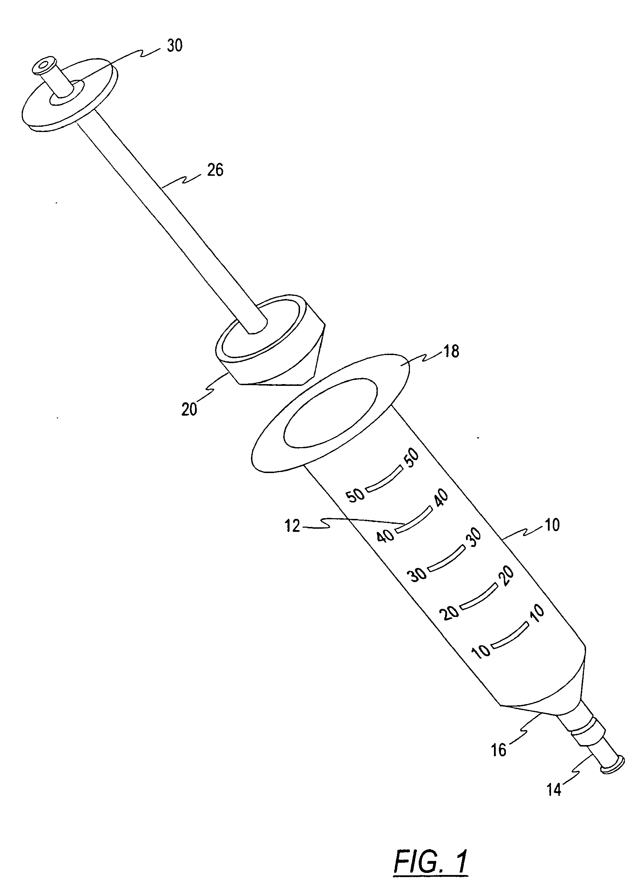

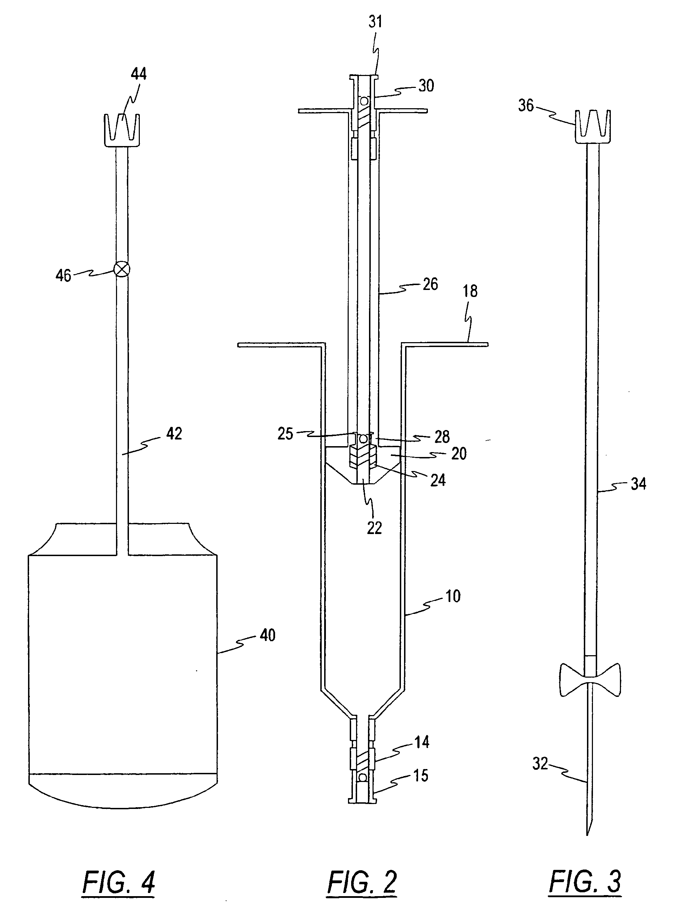

FIG. 1 shows an elongated container 10, bearing graduated marks 12 to indicate the volume of its contents (not shown). The container has a first port, shown in the drawing as including a spring-loaded ball seat valve 14, at a first end 16 and a radially-extending flange 18 at its other end. A plunger 20 fits slidably within the container and prevents blood from passing between the wall of the container and the plunger. The plunger has a through passage 22 fitted as a second port, also shown as a spring-loaded ball seat valve 24 (FIG. 2). The valve 24 could be omitted if desired, in which case, it would be necessary to temporarily seal the opening while the container is being centrifuged to separate the blood fractions. A tubular plunger rod 26 is adapted at one end 28 to engage the spring-loaded ball seat valve 24. The plunger rod has another spring-loaded ball seat valve 30 at its other end remote from the plunger. The valves 14, 24 and 30 may have a "Luer" fitting, which has a coupling 15, 25 and 31, respectively, for removably connecting to another article (for example, a hose). This type of valve is normally closed when nothing is connected to its coupling, and the valve can be opened when the protruding feature of a mating connector forces the ball away from the valve seat. It should be understood that the valves need not be the spring-loaded ball seat valves shown in the drawings, but may be other types capable of preventing the escape of the blood components and of being opened when needed, for example a valve in which a silicone plunger seals against a conical valve seat, the compression of which closes the valve until activated. More broadly, the ports may include other means for blocking flow of the blood components rather than the valves in the figures.

The container 10 and associated parts of the kit, particularly the plunger 20 and plunger rod 26, differ from conventional pliable plastic bags in being made of relatively stiff materials, for example polycarbonate, polypropylene, ABS or equivalents. It is an advantage of the present invention that, since the container walls are relatively rigid, the problems associated with separating blood fractions in a pliable bag are avoided. Moving the plunger within the container creates minimal disturbance of the separated blood layers, thereby enabling a more precise separation to be made.

FIG. 3 shows a needle 32 for taking blood from a patient connected to one end of a tube 34 which has at its other end a fitting 36 that is adapted to connect to any of the couplings 15, 25 or 31. Thus, blood may be introduced directly into container 10 through valve 14 or alternatively, blood may be directed to a separate bag for subsequent transfer into the container for separation.

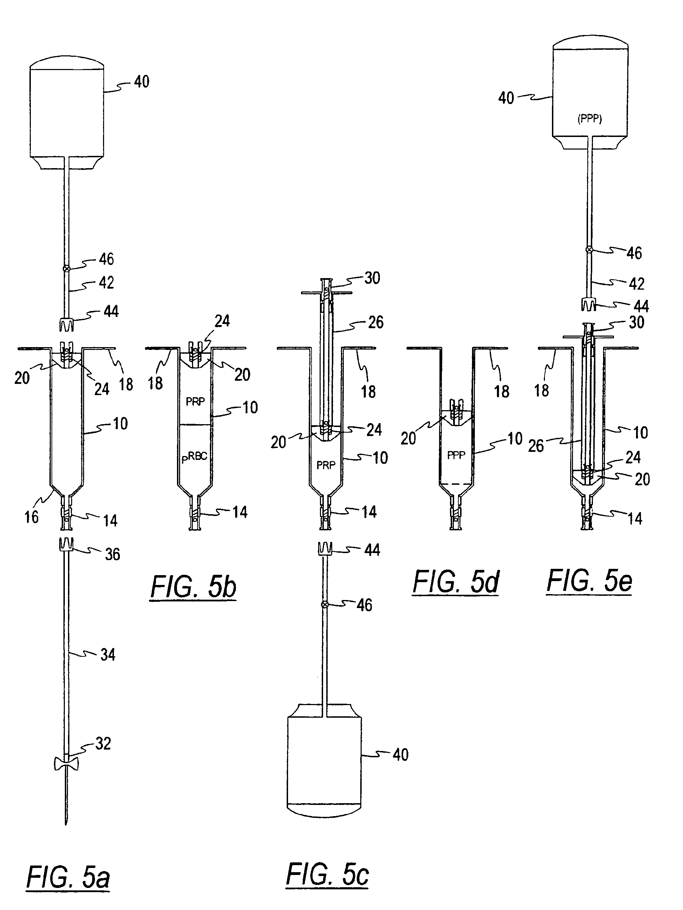

FIG. 4 shows a waste bag 40, typically but not necessarily a pliable plastic bag, connected to one end of a tube 42 which has at its other end a fitting 44 that is adapted to connect to any of the couplings 15, 25 or 31, thereby opening the associated valve. The tube 42 is fitted with a tubing clamp 46. The container 10 and associated parts shown in FIGS. 1-4, inclusive, comprise the syringe kit of this embodiment of the invention. The container 10, shorn of associated parts (plunger rod 26, needle 32 and tube 34, and waste bag 40 and tube 42) may be adapted for fitting in a centrifuge bucket (not shown) and used to separate blood into its fractions and to prepare platelet concentrate, as is described with reference to FIGS. 5a-5e, inclusive.

In FIG. 5a, the plunger 20 is at the top of the container 10, near the flange 18, the needle 32 is connected to check valve 14, which is held open, the waste bag 40 is connected to the plunger check valve 24 holding that valve open, and the clamp 46 is open. The container may be empty or it may contain a small amount of a blood anti-coagulant. When the needle 32 draws blood from a patient, the blood flows into the container under pressure from the patient, displacing air from the container 10, which air is received in the waste bag 40. When the container 10 is charged with blood, the waste bag 40 and needle 32 are decoupled from the valves 14 and 24, which are closed by the associated springs. Alternatively, the waste bag 40 is not used and instead, the plunger 20 may be used to extract blood directly from the patient in a manner similar to a conventional syringe. The container is subjected to a light spin in a centrifuge (not shown). As is shown in FIG. 5b, this step separates red blood cells (pRBC) from platelet-rich plasma (PRP). In the next step, shown in FIG. 5c, the waste bag is connected to valve 14, and the plunger rod 26 is connected to the plunger via the valve 24. The valves 14 and 24 and the waste bag clamp 46 are all open, but the valve 30 at the free end of the plunger rod is closed. The plunger rod 26 is used to push the plunger into the container, expelling the red blood cells into the waste bag 40. The container now contains primarily platelet-rich plasma. The clamp 46 is closed, and the plunger rod and the waste bag 40 are decoupled from the container, thus closing valves 14 and 24.

The container 10, shorn of the bag and plunder rod, as shown in FIG. 5d, is next subjected to a hard spin, which concentrates platelets at the bottom end 16 of the container 10, leaving platelet-poor plasma (PPP) above the platelet concentrate. The final step in the process, that of removing the platelet-poor plasma from the container, is illustrated in FIG. 5e. The plunger rod 26 is again attached to the plunger 20, and the waste bag 40 is coupled to the free end of the plunger rod through the valve 30. The valves 24 and 30 and the clamp 46 are all open, and the valve 14 is closed. The plunger is driven toward the lower end 16 of the container, and the platelet-poor plasma is forced through the passageway in plunger rod 26 and the tube 44 into the waste bag. The clamp 46 is then closed, and the waste bag 40 and, if desired, the plunger rod 26 may be removed from the container. With the waste bag removed, the plunger rod valve 30 will close, so failure to remove the plunger rod will not affect the contents of the container. The container 10 is now left with the platelet concentrate in it.

The above description covers a simple manual operation in which the syringe kit is used in a particular manner to separate blood into three fractions by successive soft and hard spins. It is also feasible to use only one "hard" spin to separate the blood in a single step into three fractions (i.e., red blood cells, plasma and an intermediate layer containing platelets). The three fractions can be separated by expelling both the plasma and red blood cells separately or simultaneously, leaving the intermediate layer for further processing. Alternatively, such manual operations can be automated.

It should be understood that the syringe kit described above is a preferred embodiment, but that modifications may be made without departing from the scope of the invention as defined by the following claims.

## Claims

1. A blood separation kit comprising:
(a) an elongated hollow container (10) having a first end and a second end and comprising a first port (14) mounted at said for end for admitting liquid to or expelling liquid from said container (10), said port (14) being closed unless opened by a fitting attached to said port;
(b) a plunger (20) movably disposed within and engaging the walls of said container (10) of (a), said plunger comprising
(1) a passageway (22) through said plunger communicating with the interior of said container of (a), and
(2) a second port (24) for expelling liquid mounted within said passageway of (b)(1), said port (24) being closed unless opened by a fitting attached to said port; and
(c) a plunger rod (26) having a passageway therein and adapted to engage said plunger of (b), said plunger rod comprising
(1) means for engaging said plunger to provide communication with the inside of the container (10) of (a) via the passageway (22) in the plunger of (b) to the passageway in said plunger road (26),
(2) a third port (30) for expelling liquid mounted at the end of said plunger rod opposite said means for engaging said plunger, said third port (30) being closed unless opened by a fitting attached to said third port.

2. A blood separation kit of Claim 1, further comprising:
(d) a needle set comprising:
(1) a hollow needle (32) for taking a sample of a patient's blood;
(2) a hollow tube (34) attached to and communicating with said needle for transferring said patient's blood to the container of (a); and
(3) a fitting (36) adapted to engage the first port of (a) and to open said first port.

3. A blood separation kit of Claims 1 or 2, further comprising:
(e) a waste bag (40) having a hollow tube (42) connected to and communicating with the interior of said bag for receiving separated blood fractions;
(f) a fitting (44) adapted to engage the first and third ports (a) and (c)(2) respectively and to open said first and third ports; and
(g) a clamp (46) mounted on the tube of (e) for opening and closing the hollow tube (42) of (e).

4. A method of separating blood and recovering a platelet-rich concentrate comprising:
(a) centrifuging an elongated blood filled container (10) having a first part and fitted with a movable plunger having a second port (24) therein; and separating said blood into platelet-rich plasma and red blood cells;
(b) displacing the red blood cells separated in (a) from said container by moving said plunger (20) towards said first port (14) and expelling said red blood cells into a waste bag (40) through tubing (42) attached to said first port;
(c) removing said waste bag (40) of (b) centrifuging said platelet-rich plasma remaining in said container (10) and separating a platelet-rich concentrate from a platelet-poor plasma;
(d) attaching a hollow plunger rod (26) having a third port (30) therein to said plunger and displacing the platelet-poor plasma separated in (c) from said container by moving said plunger towards said first port (14) and expelling said platelet-poor plasma through said second port (24) of said plunger and said third port of said plunger rod into a waste bag (40) attached to the plunger rod; and
(e) recovering the platelet-rich concentrate separated in (c) and remaining in said container.

5. A method of separating blood and recovering a platelet-rich concentrate comprising:
(a) centrifuging an elongated blood filled container (10) having a first port and fitted with a movable plunger having a second port (24) therein; and separating said blood into platelet-poor plasma, red blood cells, and platelet-rich concentrate;
(b) displacing the red blood celb separated in (a) from said container by moving said plunger (20) towards said first port (14) and expelling said red blood cells into a waste bag (40) through tubing attached to said first port (14);
(c) removing said waste bag (40) of (b) and attaching a hollow plunger rod (26) having a third port (30) herein to said plunger and displacing the platelet-poor plasma separated in (a) from said container by moving said plunger toward said first port (14) and expelling said platelet-poor plasma through said second port (24) of said plunger and said third port of said plunger rod into at a waste bag attached to the plunger rod; and
(d) recovering the platelet-rich concentrate separated in (a) and remaining in said container.

6. A blood separation kit of at least one of the claims 1 to 3, wherein said first, second, and third ports comprise valves positioned to prevent expelling blood fractions during centrifugation of blood in said container.

7. A blood separation kit of at least one of the claims 1 to 3 or 6, wherein said ports comprise Luer fittings.

## Patentansprüche

1. Kit für die Separation von Blut, umfassend:
(a) einen länglichen hohlen Behälter (10), der ein erstes Ende und ein zweites Ende aufweist und eine erste Durchlassöffnung (14) umfasst, die an dem ersten Ende montiert ist, um Flüssigkeit in den Behälter (10) aufzunehmen oder Flüssigkeit aus diesem auszustoßen, wobei die Durchlassöffnung (14) geschlossen ist, wenn sie nicht durch ein Formstück, das an der Durchlassöffnung angebracht ist, geöffnet wird;
(b) einen Kolben (20), der beweglich innerhalb der Wände des Behälters (10) von (a) angeordnet ist und mit diesen in Eingriff kommt, wobei der Kolben umfasst:
(1) einen Durchgang (22) durch den Kolben, der mit dem Inneren des Behälters von (a) in Verbindung steht, und
(2) eine zweite Durchlassöffnung (24) zum Ausstoßen von Flüssigkeit, die in dem Durchgang von (b)(1) montiert ist, wobei die Durchlassöffnung (24) geschlossen ist, wenn sie nicht durch ein Formstück, das an der Durchlassöffnung angebracht ist, geöffnet wird; und
(c) eine Kolbenstange (26), die einen Durchgang darin aufweist und dazu eingerichtet ist, mit dem Kolben von (b) in Eingriff zu kommen, wobei die Kolbenstange umfasst:
(1) eine Einrichtung zum Eingriff in den Kolben, um Verbindung mit dem Inneren des Behälters (10) von (a) über den Durchgang (22) in dem Kolben von (b) zu dem Durchgang in der Kolbenstange (26) bereitzustellen,
(2) eine dritte Durchlassöffnung (30) zum Ausstoßen von Flüssigkeit, die an dem Ende der Kolbenstange gegenüber der Einrichtung zum Eingriff in den Kolben montiert ist, wobei die dritte Durchlassöffnung (30) geschlossen ist, wenn sie nicht durch ein Formstück, das an der dritten Durchlassöffnung angebracht ist, geöffnet wird.

2. Kit für die Separation von Blut nach Anspruch 1, des Weiteren umfassend:
(d) einen Nadelsatz, umfassend:
(1) eine Hohlnadel (32) zum Entnehmen einer Blutprobe von einem Patienten;
(2) ein Hohlrohr (34), das an der Nadel angebracht ist und mit dieser in Verbindung steht, um das Blut des Patienten zu dem Behälter von (a) zu übertragen; und
(3) ein Formstück (36), das dazu eingerichtet ist, mit der ersten Durchlassöffnung von (a) in Eingriff zu kommen und die erste Durchlassöffriung zu öffnen.

3. Kit für die Separation von Blut nach den Ansprüchen 1 oder 2, des Weiteren umfassend:
(e) einen Abflussbeutel (40), bei dem ein Hohlrohr (42) an das Innere des Beutels angeschlossen ist und mit diesem in Verbindung steht, um getrennte Blutbestandteile aufzunehmen;
(f) ein Formstück (44), das dazu eingerichtet ist, mit der ersten bzw. der dritten Durchlassöffnung ((a) bzw. (c)(2)) in Eingriff zu kommen und die erste und die dritte Durchlassöffnung zu öffnen; und
(g) eine Klemme (46), die an dem Rohr von (e) montiert ist, um das Hohlrohr (42) von (e) zu öffnen und zu schließen.

4. Verfahren zur Separation von Blut und zum Gewinnen eines blutplättchenreiches Konzentrats, umfassend:
(a) Zentrifugieren eines länglichen blutgefüllten Behälters (10), der eine erste Durchlassöffnung aufweist und mit einem beweglichen Kolben (20) mit einer zweiten Durchlassöffnung (24) darin ausgestattet ist, und Trennen des Blutes in blutplättchenreiches Plasma und rote Blutkörperchen;
(b) Verdrängen der in (a) getrennten roten Blutkörperchen aus dem Behälter durch Bewegen des Kolbens (20) in Richtung der ersten Durchlassöffnung (14) und Ausstoßen der roten Blutkörperchen über Rohrleitungen (42), die an der ersten Curchlassöffnullg angebracht sind, in einen Abflussbeutel (40);
(c) Entnehmen des Abflussbeutels (40) von (b), Zentrifugieren des blutplättchenreichen Plasmas, das in dem Behälter (10) verblieben ist, und Trennen eines blutplättchenreichen Konzentrats von einem blutplättchenarmen Plasma;
(d) Anbringen einer hohlen Kolbenstange (26) mit einer dritten Durchlassöffnung (30) darin an dem Kolben und Verdrängen des in (c) getrennten blutplättchenarmen Plasmas aus dem Behälter durch Bewegen des Kolbens in Richtung der ersten Durchlassöffnung (14) und Ausstoßen des blutkörperchenarmen Plasmas über die zweite Durchtassöffnung (24) des Kolbens und die dritte Durchlassöffnung der Kolbenstange in einen Abflussbeutel (40), der an der Kolbenstange angebracht ist; und
(e) Gewinnen des blutplättchenreichen Konzentrats, das in (c) getrennt wurde und in dem Behälter verblieben ist.

5. Verfahren zur Separation von Blut und zum Gewinnen eines blutplättchenreiches Konzentrats, umfassend:
(a) Zentrifugieren eines länglichen blutgefüilten Behälters (10), der eine erste Durchlassöftnung aufweist und mit einem beweglichen Kolben (20) mit einer zweiten Durchtassöffnung (24) darin ausgestattet ist, und Trennen des Blutes in blutplättchenarmes Plasma, rote Blutkörperchen und blutplättchenreiches Konzentrat;
(b) Verdrängen der in (a) getrennten roten Blutkörperchen aus dem Behälter durch Bewegen des Kolbens (20) in Richtung der ersten Durchlassöffnung (14) und Ausstoßen der roten Blutkörperchen über Rohrleitungen (42), die an der ersten Durchlassöffnung (14) angebracht sind, in einen Abflussbeutel (40);
(c) Entnehmen des Abflussbeutels (40) von (b) und Anbringen einer hohlen Kolbenstange (26) mit einer dritten Durchlassöffnung (30) darin an dem Kolben und Verdrängen des in (a) getrennten biutplättchenarmen Plasmas aus dem Behälter durch Bewegen des Kolbens in Richtung der ersten Durchlassöffnung (14) und Ausstoßen des blutkörperchenarmen Plasmas über die zweite Durchlassöffnung (24) des Kolbens und die dritte Durchlassöffnung der Kolbenstange in einen Abflussbeutel, der an der Kolbenstange angebracht ist; und
(e) Gewinnen des blutplättcherrreicrien Konzentrats, das in (a) getrennt wurde und in dem Behälter verblieben ist.

6. Kit für die Separation von Blut nach wenigstens einem der Ansprüche 1 bis 3, wobei die erste, zweite und dritte Durchlassöffnung Ventile umfassen, die so positioniert sind, dass das Ausstoßen von Blutbestandteilen während der Zentrifugierung des Blutes in dem Behälter verhindert wird.

7. Kit für die Separation von Blut nach wenigstens einem der Ansprüche 1 bis 3 oder 6, wobei die Durchlassöffnungen Luer-Formstücke umfassen.

## Revendications

1. Trousse de séparation de sang, comprenant :
(a) un conteneur (10) allongé et creux ayant une première extrémité et une seconde extrémité et comprenant un premier orifice (14) placé au niveau de ladite première extrémité pour admettre du liquide audit conteneur (10) ou pour expulser du liquide de celui-ci, ledit orifice (14) étant fermé à moins d'être ouvert par un raccord rattaché audit orifice ;
(b) un piston (20) disposé de manière mobile dans ledit conteneur (10) du point (a) et s'engageant contre les parois de celui-ci, ledit piston comprenant :
(1) un passage (22) à travers ledit piston communiquant avec l'intérieur dudit conteneur du point (a), et
(2) un deuxième orifice (24) servant à l'expulsion du liquide, placé à l'intérieur dudit passage du point (b)(1), ledit orifice (24) étant fermé à moins d'être ouvert par un raccord rattaché audit orifice ; et
(c) une tige de piston (26) contenant un passage et adaptée pour s'engager avec ledit piston du point (b), ladite tige de piston comprenant :
(1) un moyen d'engagement avec ledit piston pour permettre la communication avec l'intérieur du conteneur (10) du point (a) via le passage (22) du piston du point (b) et le passage de ladite tige de piston (26),
(2) un troisième orifice (30) servant à l'expulsion du liquide, placé à l'extrémité de ladite tige de piston à l'opposé dudit moyen d'engagement avec ledit piston, ledit troisième orifice (30) étant fermé à moins d'être ouvert par un raccord rattaché audit troisième orifice.

2. Trousse de séparation de sang selon la revendication 1, comprenant en outre :
(d) un ensemble d'aiguille comprenant :
(1) une aiguille creuse (32) permettant de prélever un échantillon de sang d'un patient ;
(2) un tube creux (34) rattaché à ladite aiguille et communiquant avec celle-ci pour permettre le transfert du sang dudit patient dans le conteneur du point (a) ; et
(3) un raccord (36) adapté pour s'engager avec le premier orifice du point (a) et pour ouvrir ledit premier orifice.

3. Trousse de séparation de sang selon la revendication 1 ou 2, comprenant en outre :
(e) un sac à déchets (40) ayant un tube creux (42) relié avec l'intérieur dudit sac et communiquant avec celui-ci pour recevoir les fractions sanguines séparées ;
(f) un raccord (44) adapté pour s'engager avec les premier et troisième orifices (a) et (c)(2), respectivement, et pour ouvrir lesdits premier et troisième orifices ; et
(g) un dispositif de serrage (46) placé sur le tube du point (e) pour ouvrir et fermer le tube creux (42) du point (e).

4. Procédé de séparation de sang et de récupération d'un concentré riche en plaquettes, comprenant :
(a) la centrifugation d'un conteneur (10) allongé rempli de sang ayant un premier orifice et muni d'un piston mobile (20) contenant un deuxième orifice (24), et la séparation dudit sang en plasma riche en plaquettes et en globules rouges ;
(b) le déplacement des globules rouges séparés dans l'étape (a) depuis ledit conteneur en repoussant ledit piston (20) vers ledit premier orifice (14) et l'expulsion desdits globules rouges dans un sac à déchets (40) à travers une tubulure (42) rattachée audit premier orifice ;
(c) le retrait dudit sac à déchets (40) du point (b), la centrifugation dudit plasma riche en plaquettes restant dans ledit conteneur (10) et la séparation d'un concentré riche en plaquettes et d'un plasma pauvre en plaquettes ;
(d) le rattachement d'une tige de piston creuse (26) contenant un troisième orifice (30) audit piston et le déplacement du plasma pauvre en plaquettes séparé dans l'étape (c) depuis ledit conteneur en repoussant ledit piston vers ledit premier orifice (14) et l'expulsion dudit plasma pauvre en plaquettes à travers ledit deuxième orifice (24) dudit piston et ledit troisième orifice de ladite tige de piston dans un sac à déchets (40) rattaché à la tige de piston ; et
(e) la récupération du concentré riche en plaquettes séparé dans l'étape (c) et restant dans ledit conteneur.

5. Procédé de séparation de sang et de récupération d'un concentré riche en plaquettes, comprenant :
(a) la centrifugation d'un conteneur (10) allongé rempli de sang ayant un premier orifice et muni d'un piston mobile contenant un deuxième orifice (24), et la séparation dudit sang en plasma pauvre en plaquettes, en globules rouges et en concentré riche en plaquettes ;
(b) le déplacement des globules rouges séparés dans l'étape (a) depuis ledit conteneur en repoussant ledit piston (20) vers ledit premier orifice (14) et l'expulsion desdits globules rouges dans un sac à déchets (40) à travers une tubulure rattachée audit premier orifice (14) ;
(c) le retrait dudit sac à déchets (40) du point (b) et le rattachement d'une tige de piston creuse (26) contenant un troisième orifice (30) audit piston et le déplacement du plasma pauvre en plaquettes séparé dans l'étape (a) depuis ledit conteneur en repoussant ledit piston vers ledit premier orifice (14) et l'expulsion dudit plasma pauvre en plaquettes à travers ledit deuxième orifice (24) dudit piston et ledit troisième orifice de ladite tige de piston dans un sac à déchets rattaché à la tige de piston ; et
(d) la récupération du concentré riche en plaquettes séparé dans l'étape (a) et restant dans ledit conteneur.

6. Trousse de séparation de sang selon l'une au moins des revendications 1 à 3, dans laquelle lesdits premier, deuxième et troisième orifices comprennent des valves positionnées de manière à empêcher l'expulsion de fractions sanguines durant la centrifugation du sang dans ledit conteneur.

7. Trousse de séparation de sang selon l'une au moins des revendications 1 à 3 ou 6, dans laquelle lesdits orifices comprennent des raccords Luer.
